# EUROPEAN PATENT APPLICATION

(11) **EP 4 692 323 A1**
(43) Date of publication of application: **11.02.2026**
(21) Application number: 24872463.5
(22) Date of filing: 26.09.2024
(51) Int. Cl.: C12N 5/0775, A61K 35/28, A61P 17/02, A61P 19/08, C12N 5/02

(54) **METHOD FOR PRODUCING EXOSOME-CONTAINING LIQUID DERIVED FROM MESENCHYMAL STEM CELLS, AND PHARMACEUTICAL PRODUCT CONTAINING EXOSOME-CONTAINING LIQUID**

(30) Priority: 27.09.2023 JP 2023164070; 01.06.2024 WO PCT/JP2024/020128
(71) Applicant: Meis Technology Co., Ltd, Nagoya-shi, Aichi 460-0003 (JP); Biolabo Co., Ltd, Amami-shi, Kagoshima 894-0026 (JP)
(72) Inventor: YAMAMOTO, Tokunori, Nagoya-shi, Aichi 464-0858 (JP); KUBOSHIMA, Hajime, Amami-shi, Kagoshima 894-0026 (JP); TAKAGI, Souichi, Amami-shi, Kagoshima 894-0026 (JP)
(74) Representative: Algemeen Octrooi- en Merkenbureau B.V.
(86) International application number: PCT/JP2024/034550
(87) International publication number: WO 2025/070679

(57) **Abstract**

To provide a method for producing a high concentration exosome-containing solution in a large amount and easily.

The present inventors have found that an exosome-containing solution having a high concentration can be easily produced in a large amount by disrupting mesenchymal stem cells. Specifically, the method includes: (1) a step of disrupting mesenchymal stem cells; (2) a step of stirring the disrupted liquid obtained in the step (1) with a stirrer or a vortex mixer; and (3) a step of filtering the disrupted solution after the stirring treatment through a filter. The exosome-containing solution produced in the present invention is cell-free, and thus is easy to prepare and handle, and long-term storage by freezing is also possible. In addition, no additive is added, and thus there is no need to worry about the influence of the additive on the exosomes, and the advantages in research and clinical applications are extremely large.

## Description

### TECHNICAL FIELD

The present invention relates to a method for producing an exosome-containing solution derived from mesenchymal stem cells and a pharmaceutical including an exosome-containing solution.

### BACKGROUND ART

Mesenchymal stem cells are capable of self-proliferation and can differentiate into various cells including chondrocytes, osteocytes, myocytes, adipocytes, and the like. Recently, various factors (paracrine factors) secreted by mesenchymal stem cells have attracted attention rather than differentiation potency of mesenchymal stem cells themselves. It has been found that mesenchymal stem cells secrete cytokines and chemokines, growth factors, and exosomes having an inflammation suppressing effect, an angiogenesis promoting effect, and a cell proliferation promoting effect, and promote tissue repair through the paracrine effect (see, for example, Non Patent Literature 1).

An exosome is a vesicle composed of a lipid bilayer, and is a substance secreted from a cell (originating cell) to the outside of a cell. Exosomes encompass various proteins, lipids, mRNAs, microRNAs, cell-specific components reflecting biological functions from the originating cell, and the like. Exosomes can fuse with cells distant from the originating cell to release the inclusion, and thus they are considered to play a functional role in mediating cell-cell communication and cellular immunity. Therefore, in recent years, therapeutic effects using exosomes on various diseases have been studied. For example, utilization in drug transmission systems for delivering various therapeutic agents to target cells has been studied (see, for example, Non Patent Literatures 2 and 3), and utilization as biomarkers of neurological diseases such as Alzheimer's disease has been studied (see, for example, Non Patent Literature 4).

The nature of exosomes varies depending on the type of the originating cell. In particular, it is known that exosomes derived from mesenchymal stem cells exhibit regenerative medical therapeutic effects possessed by stem cells, and research on treatment using exosomes has been actively conducted in the fields of wound and fracture healing and regenerative medicine.

In order to use exosomes in clinical and research, it is necessary to produce a large amount of high-quality exosomes. Examples of the method for mass-producing exosomes include a method using a "composition for promoting stem cell-derived exosome production" in which the number of stem cell-derived exosomes is increased by culturing stem cells in a cell culture medium including a specific compound (see, for example, Patent Literature 1), and a method using a cell population in which 80% or more of cells are specific marker proteins such as CD73⁺ in a cell population including precursor cells of mesenchymal stem cells (see, for example, Patent Literature 2).

### CITATIONS LIST

### PATENT LITERATURE

Patent Literature 1: JP 2022-533277 T
Patent Literature 2: JP 2022-542158 T

### NON-PATENT LITERATURE

Non Patent Literature 1: Chamberlain G., Fox J., Ashton B., Middleton J., "Concise review: mesenchymal stem cells: their phenotype, differentiation capacity, immunological features, and potential for homing." Stem Cells. 25 (2007) 2739-2749
Non Patent Literature 2: Emmanouilidou E. et al., "Cell-produced alpha-synuclein is secreted in a calcium-dependent manner by exosomes and impacts neuronal survival", J Neurosci. 2010 May 19; 30 (20): 6838-51
Non Patent Literature 3: Ian Fyfe, "Exosomes can spread toxic AD pathology" Nat Rev Neurol. 2018 Aug; 14(8): 451
Non Patent Literature 4: Yamayoshi A., et al., "Development of Novel Drug Delivery System Targeting Exosomal microRNA" The Pharmaceutical Society of Japan, Vol. 140, No. 5, 625-631 (2020)

### SUMMARY OF INVENTION

### TECHNICAL PROBLEMS

The type of cells from which exosomes are derived and conditions during production affect the quality. Therefore, it is preferable not to add additives, reagents, and the like, but in the technique described in Patent Literature 1, exosomes are produced by adding a compound that is not in vivo, such as exendin-4, and thus it is not clear how much adverse effect is exerted on cells and exosomes. In addition, there is the problem that a cleaning step is required to clean the additive, and the work is troublesome. In addition, in the technique described in Patent Literature 2, a precursor cell of a mesenchymal stem cell is essential, and the method for producing the precursor cell is problematic in that it takes time and labor.

Therefore, the present invention is directed to providing a method for producing an exosome-containing solution in a large amount and easily at a high concentration without adding an artificial compound such as an exosome production promoter.

### SOLUTIONS TO PROBLEMS

In order to solve the above problems, the present inventors have focused on mesenchymal stem cells, investigated that a large amount of an exosome-containing solution having a high concentration can be easily produced by disrupting cells, and completed the present invention.

That is, the present invention has the following configuration.
[1] A method for producing an exosome-containing solution derived from mesenchymal stem cells, the method including:
   (1) a step of disrupting mesenchymal stem cells;
   (2) a step of stirring the disrupted solution obtained in the step (1) using a stirrer or a vortex mixer; and
   (3) a step of filtering the disrupted solution after the stirring treatment through a filter.
[2] The method for producing an exosome-containing solution according to [1], wherein the mesenchymal stem cells are adipose tissue-derived mesenchymal stem cells.
[3] The method for producing an exosome-containing solution according to [1] or [2], wherein the step (1) is performed by freeze-thawing or ultrasonic waves.
[4] The method for producing an exosome-containing solution according to [1] or [2], wherein the disrupted solution is stirred at 100 to 3,500 rpm for 1 to 30 minutes using a vortex mixer in the step (2).
[5] A pharmaceutical including, as an active ingredient, an exosome-containing solution produced by the method according to [1] or [2].
[6] A method for promoting recovery from fatigue, wherein the pharmaceutical according to [5] is administered by intravenous injection.
[7] A supplement for non-human mammals, including, as an active ingredient, an exosome-containing solution produced by the method according to [1] or [2].

### ADVANTAGEOUS EFFECTS OF INVENTION

The production method of the present invention can easily produce an exosome-containing solution having a high concentration in a large amount. An expensive machine such as an ultracentrifuge is not required, and thus the production cost can also be reduced. In addition, not living cells but cell fragments are used for production, and thus the obtained exosome-containing solution is of cell free. Therefore, the exosome-containing solution of the present invention has a low risk of infection and carcinogenesis derived from cells, and there is no risk of causing blockage in microvessels when administered as a pharmaceutical. In addition, it is not necessary to perform cell culture with precise timing of use like living cells, preparation and handling are easy, and long-term storage by freezing is also possible. Further, according to the present invention, it is not necessary to add an additive such as an exosome production promoter. There is no need to worry about the influence of additives on exosomes, and thus research and clinical advantages are extremely large. In addition, pharmaceuticals and supplements including the exosome-containing solution of the present invention as an active ingredient can be expected to have a fatigue recovery promoting effect.

### BRIEF DESCRIPTION OF DRAWING

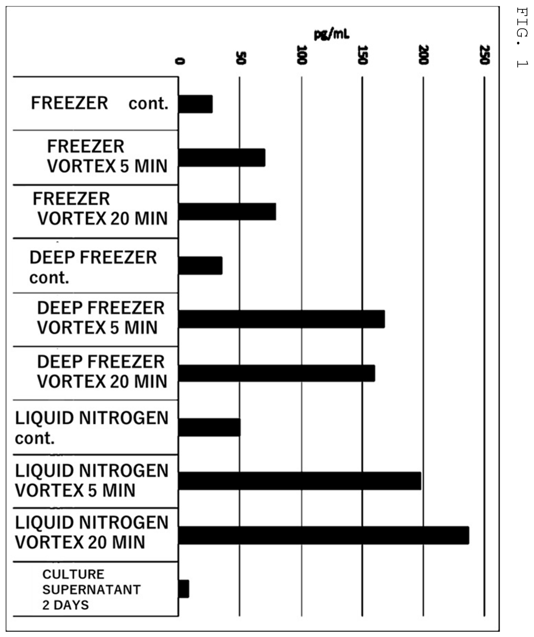

Fig. 1 is a measurement result of an exosome amount in an exosome-containing solution.

### DESCRIPTION OF EMBODIMENTS

### <Exosome>

In the present invention, the "exosome" is an endoplasmic reticulum composed of a lipid bilayer, the endoplasmic reticulum being secreted from the cell to the outside of the cell, and the inside of the lipid bilayer membrane includes proteins, lipids, nucleic acids (miRNA, mRNA, DNA, and the like), and the like.

Exosomes are endoplasmic reticula that are secreted from the cells to the outside of the cells. The formation of exosomes begins with the formation of an early endosome by endocytosis, involving receptors present in the cell membrane. The early endosome translocates into the late endosome, which invaginates inward to form intraluminal membrane vesicles (ILVs). An endosome (multivesicular body: MVB) containing a large number of ILVs finally fuses with the cell membrane and the ILVs are released from the cell.

The present inventors have focused on mesenchymal stem cells themselves that secrete exosomes, and have succeeded in extracting exosomes included in the cells before being secreted in addition to the exosomes secreted by disrupting the cells.

### <Mesenchymal stem cell>

In the present invention, the mesenchymal stem cell which secretes exosomes is a cell belonging to the mesenchymal system, and is known to have pluripotency and self-replication potency, and to have differentiation potency into connective tissue cells such as osteocytes, chondrocytes, and adipocytes, and also into nerve cells and cardiomyocytes.

Examples of the tissue including mesenchymal stem cells include adipose tissue, umbilical cord, bone marrow, umbilical cord blood, endometrium, placenta, amniotic membrane, chorionic membrane, decidua, dermis, skeletal muscle, periosteum, dental follicle, periodontal ligament, dental pulp, and tooth germ, and adipose tissue-derived mesenchymal stem cells, umbilical cord blood-derived mesenchymal stem cells, bone marrow-derived mesenchymal stem cells, or dental pulp-derived mesenchymal stem cells are preferable, and among these, adipose tissue-derived mesenchymal stem cells are preferable in that easy collection is possible.

Preparation of adipose tissue-derived mesenchymal stem cells, umbilical cord blood-derived mesenchymal stem cells, bone marrow-derived mesenchymal stem cells, and dental pulp-derived mesenchymal stem cells follows a conventional method. Herein, a method for preparing adipose-derived mesenchymal stem cells, which are preferred cells, will be specifically described.

The biological origin of the mesenchymal stem cells in the present invention is not particularly limited, and includes a mammal other than humans (including pet animals, livestock, and experimental animals. Specifically, for example, a monkey, a pig, a cow, a horse, a goat, a sheep, a dog, a cat, a camel, a mouse, a rat, a guinea pig, and a hamster), in addition to humans. When the exosome-containing solution of the present invention is administered as a pharmaceutical, it is preferable to collect mesenchymal cells from the same solid as the administration subject (recipient) (autologous). However, the use of mesenchymal stem cells of the same type of animal (allogeneic) or mesenchymal stem cells of a different type of animal is not hindered.

### <Adipose tissue-derived mesenchymal stem cells>

Adipose tissue-derived mesenchymal stem cells (referred to as adipose-derived stem cells: ADSCs (ASCs), adipose-derived regeneration cells: ADRCs, adipose tissue-derived mesenchymal stem cells: AT-MSCs, AD-MSCs, or the like, hereinafter, may be simply referred to as "ADSCs") are a type of somatic stem cells, and are stem cells included in adipose tissue. Adipose tissue-derived mesenchymal stem cells also have self-replication potency and pluripotency, and are known to be capable of differentiating into not only fat but also various cells such as bone, cartilage, nerve, muscle, myocardium, blood vessel, hepatocyte, and pancreatic islet cells.

### <Method for preparing adipose tissue-derived mesenchymal stem cells>

In the present invention, as long as ADSCs maintain pluripotency, cells obtained by culturing (including subculture) the somatic stem cells also correspond to ADSCs. Typically, ADSCs are prepared in an "isolated state" as cells constituting a cell population (including cells other than ADSCs derived from adipose tissue) using adipose tissue separated from a living body as a starting material. Herein, the "isolated state" means a state of being taken out from its original environment (that is, a state in which a part of a living body is constituted), that is, a state of being present in a state different from an original state due to artificial operation.

Preparation of ADSCs in the present invention only has to be performed according to a conventional method. ADSCs are widely used in various applications, and thus those skilled in the art can also prepare ADSCs with reference to literatures and documents. A cell distributed from a public cell bank, a commercially available cell, or the like may be used. Hereinafter, as an example of the method for preparing cells, a method for preparing adipose tissue-derived mesenchymal stem cells (one example) will be described.

ADSCs are prepared through steps such as separation, washing, concentration, and culture of stem cells from adipose matrix. The preparation method of ADSCs is not particularly limited. For example, ADSCs can be prepared according to known methods (see, Fraser JK et al. (2006), Fat tissue: an under appreciated source of stem cells for biotechnology. Trends in Biotechnology; Apr; 24 (4): 150-4. Epub 2006 Feb 20. Review.; Zuk PA et al. (2002), Human adipose tissue is a source of multipotent stem cells. Molecular Biology of the Cell; Dec; 13 (12): 4279-95.; Zuk PA et al. (2001), Multilineage cells from human adipose tissue: implications for cell-based therapies. Tissue Engineering; Apr; 7 (2): 211-28.; and the like). In addition, a device for preparing ADSCs from adipose tissue (for example, a Celution (registered trademark) device (Cytori Therapeutics, Inc., San Diego, USA)) is also commercially available, and ADSCs may be prepared using the device. When the device is used, a cell population including ADSCs can be separated from adipose tissue (K. Lin. Et al. Cytotherapy (2008) Vol. 10, No. 4, 417-426). Hereinafter, a specific example of a preparation method of ADSCs will be described.

### (1) Preparation of cell population from adipose tissue

Adipose tissue is collected from a human and a non-human mammal by means such as excision and aspiration. The non-human mammal may be any animal as long as it is the animal described above, and any pet animal, livestock, or laboratory animal is acceptable. In addition, the age and sex of the organism are not particularly limited. In a human, ADSCs can also be prepared from tissue pieces sucked by liposuction surgery at the time of cosmetic surgery, or removed adipose tissue included in tissue removed from a living body at the time of surgery or the like. ADSCs are present around large blood vessels and thus can be obtained more from excised adipose tissue than from lipoaspirate liquid. On the other hand, when the stem cells are prepared from the lipoaspirate liquid, the surgical scar is small, and the burden on the donor is small.

Examples of the adipose tissue include subcutaneous fat, visceral fat, intramuscular fat, and intermuscular fat. Among these, subcutaneous fat can be collected significantly easily under local anesthesia, and thus the burden on the donor at the time of collection is small, and it can be said to be a preferable cell source. Typically, one type of adipose tissue is used, but two or more types of adipose tissue can be used in combination. In addition, adipose tissue (not necessarily the same type of adipose tissue) collected in a plurality of times may be mixed and used for the subsequent operations.

The collection amount of adipose tissue can be determined in consideration of the type of donor and the type of tissue, or the amount of ADSCs required, and is, for example, about 0.5 to 500 g. However, in consideration of the burden on the donor, the amount collected at a time is preferably about 3 to 20 g or less. The collected adipose tissue is subjected to the following enzyme treatment after being subjected to removal of blood components attached thereto and fragmentation as necessary. The blood component can be removed by washing the adipose tissue in an appropriate buffer or culture solution.

The enzyme treatment is performed by digesting the adipose tissue with an enzyme such as collagenase, trypsin, or dispase. Such enzyme treatment only has to be performed by methods and conditions known to those skilled in the art (see, R. I. Freshney, Culture of Animal Cells: A Manual of Basic Technique, 4th Edition, A John Wiley & Sones Inc., Publication). The cell population obtained by the above enzyme treatment includes, for example, pluripotent stem cells, endothelial cells, stromal cells, blood cells, precursor cells thereof. The type, ratio, and the like of the cells constituting the cell population depend on the origin and type of the used adipose tissue.

### (2) Acquisition of precipitated cell populations (SVF fractions: stromal vascular fractions)

The cell population is subsequently subjected to centrifugation. The sediment obtained by centrifugation is collected as a precipitated cell population (also referred to as "SVF fraction" herein). The conditions of the centrifugal treatment vary depending on the type and amount of cells, and are, for example, 1 to 10 minutes and 800 to 1,500 rpm. Prior to the centrifugal treatment, it is preferable that the cell population after the enzyme treatment is subjected to filtration or the like to remove enzyme-undigested tissue and the like included therein. The "SVF fraction" obtained herein includes ADSCs. The type, ratio, and the like of cells constituting the SVF fraction depend on the origin and type of the adipose tissue used, the conditions of enzyme treatment, and the like. In addition, International Publication No. WO2006/006692 A1 discloses characteristics of the SVF fraction.

### (3) Selective culture of adherent cells (ADSCs) and collection of cells

The SVF fraction includes other cell components (endothelial cells, stromal cells, blood cells, precursor cells thereof, and the like) in addition to ADSCs. Therefore, in one embodiment of the present invention, the following selective culture is performed to remove unnecessary cell components from the SVF fraction. Then, the cells thus obtained are used in the present invention as ADSCs. First, the SVF fraction is suspended in an appropriate medium, and then seeded on a culture dish and cultured overnight. Floating cells (non-adherent cells) are removed by medium exchange. Thereafter, the culture is continued while appropriately replacing the culture medium (for example, once every 2 to 4 days). If necessary, subculture is performed. The passage number is not particularly limited, and from the viewpoint of maintaining pluripotency and proliferation capability, it is not preferable to repeat the passage excessively (it is preferable to keep the passage number up to approximately 6 passages). As the culture medium, a normal culture medium for animal cell culture can be used. For example, Dulbecco's modified Eagle's Medium (DMEM) (e.g., NISSUI PHARMACEUTICAL CO., LTD.), α-MEM (e.g., Dainippon Pharmaceutical Co., Ltd.), DMEM: Ham's F12 mixed medium (1:1) (e.g., Dainippon Pharmaceutical Co., Ltd.), Ham's F12 medium (e.g., Dainippon Pharmaceutical Co., Ltd.), MCDB201 medium (Research Institute for the Functional Peptides Co., Ltd.), or the like can be used. A medium to which serum (fetal bovine serum, human serum, sheep serum, or the like) or a serum replacement (Knockout serum replacement (KSR) or the like) is added may be used. The amount of the serum added or serum alternative added can be set, for example, within a range of 5 to 30% (v/v).

The above operation causes the adherent cells to selectively survive and proliferate. Subsequently, the proliferated cells are collected. The collection operation only has to be performed according to a conventional method, and for example, cells after enzyme treatment (trypsin or dispase treatment) can be easily collected by peeling the cells with a cell scraper, a pipette, or the like. In addition, when sheet culture is performed using a commercially available temperature sensitive culture dish or the like, it is also possible to collect the cells as they are in the form of a sheet without enzyme treatment. Using the cells (ADSC) collected in this way allows a cell population including ADSCs with high purity to be prepared.

### (4) Low serum culture (selective culture in low serum medium) and collection of cells

In one embodiment of the present invention, the following low serum culture is performed instead of the operation of (3) or after the operation of (3). Then, the cells thus obtained are used in the present invention as ADSCs. In low serum culture, the SVF fraction (when this step is performed after (3), the cells collected in (3) are used) is cultured under low serum conditions to selectively proliferate pluripotent stem cells of interest (i.e., ADSCs). The low serum culture method requires only a small amount of serum, and thus when ADSCs obtained by the method of the present invention are used for therapeutic purposes, the serum of the subject (patient) can be used. That is, culture using autologous serum becomes possible. Herein, the "low serum condition" is a condition in which 5% (v/v) or less of serum is included in a culture medium. Preferably, the cells are cultured in a culture solution including 2% (v/v) or less of serum. More preferably, the cells are cultured in a culture solution including 2% (v/v) or less of serum and 1 to 100 ng/mL of fibroblast growth factor -2 (bFGF). A serum-free medium may be used.

The serum is not limited to fetal bovine serum, and human serum, sheep serum, and the like can be used. When the activated sperm obtained by the method of the present invention is used for treatment of humans, human serum is preferably used, and serum of the treatment subject (that is, autologous serum) is more preferably used. As the culture medium, a normal culture medium for animal cell culture can be used under the condition that the amount of serum contained at the time of use is low. For example, Dulbecco's modified Eagle's Medium (DMEM) (e.g., NISSUI PHARMACEUTICAL CO., LTD.), α-MEM (e.g., Dainippon Pharmaceutical Co., Ltd.), DMEM: Ham's F12 mixed medium (1:1) (e.g., Dainippon Pharmaceutical Co., Ltd.), Ham's F12 medium (e.g., Dainippon Pharmaceutical Co., Ltd.), MCDB201 medium (Research Institute for the Functional Peptides Co., Ltd.), or the like can be used.

Culturing by the above method allows ADSCs to be selectively proliferated. In addition, ADSCs proliferating under the above culture conditions have high proliferation activity, and thus the number of cells required for the present invention can be easily prepared by subculture. International Publication No. WO2006/006692 A1 discloses characteristics of cells that selectively proliferate by culturing the SVF fraction under low-serum conditions. Subsequently, cells selectively proliferated by the above-described low serum culture are collected. The collection operation only has to be performed in the same manner as in the case of (3) above. Using the collected ADSCs can provide a cell population containing ADSCs with high purity.

In the above method, cells proliferated by culturing the SVF fraction in low serum are used, but cells proliferated by directly culturing a cell population obtained from adipose tissue in low serum (without performing centrifugation treatment for obtaining the SVF fraction) may be used as ADSCs. That is, in one embodiment of the present invention, cells proliferated when a cell population obtained from adipose tissue is cultured in low serum are used as ADSCs. In addition, instead of the pluripotent stem cells obtained by selective culture (the above (3) and (4)), the SVF fraction (containing adipose tissue-derived mesenchymal stem cells) may be used as it is. Herein, "use as it is" means use in the present invention without undergoing selective culture.

### <Method for preparing exosome-containing solution>

### (Step (1): step of disrupting ADSCs)

For disruption of ADSCs, common cell disruption methods can be used. For example, a treatment method using freeze-thawing (treatment of freezing and then thawing), ultrasonic waves, French press, mortar, homogenizer, glass beads, or the like can be used. In addition, as the cells to be subjected to the disrupting treatment, not only living cells but also dead cells or damaged cells may be used. Among the above disrupting treatment, freeze-thawing treatment and ultrasonic treatment are preferable. In particular, freeze-thawing treatment is particularly preferable from the viewpoint of being simple, being capable of avoiding contamination due to contact between a machine and a cell, and being hygienic. In the case of disrupting by freeze-thawing, preferable conditions described later can be used. In the case of disrupting by ultrasonic treatment, it is preferable to perform disrupting treatment using unfrozen cells, and it is preferable to repeatedly perform treatment for a short time while cooling the cell suspension in ice because denaturation and aggregation of proteins are often caused by the influence of heat emitted from the device. Specifically, it is preferable to repeat disrupting for 5 to 15 seconds and pausing for 10 to 30 seconds at an output of 200 W to 300 W a plurality of times.

Incidentally, freeze-thawing is preferably repeated in order to sufficiently dissolve the cells because the cells expand in the freezing process to form ice crystals, and the ice crystals break the cells, whereby the cells are dissolved during thawing. Specifically, the freeze-thawing treatment is preferably repeated 1 to 5 times. It is more preferable to repeat 2 to 4 times. Examples of the freezing means include a freezer, a deep freezer, and liquid nitrogen. The freezing temperature in the freeze-thawing treatment is not particularly limited as long as it is a temperature at which the entire cell population can be frozen, and is preferably -20°C to -196°C, more specifically, the upper limit of the freezing temperature is preferably -20°C or lower, more preferably -30°C or lower, and still more preferably -70°C or lower.

The cooling time is not particularly limited as long as the entire cell population can be frozen, and in the case of using a freezer, it is preferable to place the cells in the freezer for 1 hour or longer, preferably 10 hours or longer to sufficiently cool the cells. The upper limit of the cooling time is not particularly limited, and is preferably 72 hours or shorter, more preferably 48 hours or shorter, and still more preferably 12 hours or shorter. It is efficient to put the cell population in a refrigerator or freezer before going home, cool it overnight, and work on the next day. When liquid nitrogen is used, a container including cells may be directly stored or cells may be stored in a cryopreservation container, and the container may be immersed in liquid nitrogen and cooled.

Thawing of frozen cells can be performed by any means. For example, the frozen cells can be fed to a water bath, an incubator, a refrigerator, a thermostatic bath, or the like having a temperature higher than the freezing temperature. Specifically, it is possible to adopt melting by standing overnight in a refrigerator at 5°C or lower, melting by double boiling (for example, 35 to 40°C), melting at room temperature, and the like. The concentration of the cell suspension used for disrupting is preferably 1 × 10⁴ to 1 × 10⁸ cells/mL of ADSCs. It is further preferably 10 × 10⁴ to 1000 × 10⁴ cells/mL, and more preferably 10 × 10⁴ to 500 × 10⁴ cells/mL of ADSCs. The concentration is easy to work with, and a sufficient amount of the exosome-containing solution can be taken by one work.

### (Step (2): step of stirring disrupted solution)

The disrupted solution is put in a container such as a test tube and stirred using a stirring bar (stirrer) or a vortex mixer. When a stirring bar is used, an operation such as washing of the stirring bar is required thereafter, and there is a concern about loss of exosomes due to adhesion of the disrupted solution to the stirring bar, and thus in order to increase production efficiency, stirring treatment by a vortex mixer is preferable. The number of revolutions by the vortex mixer for obtaining an exosome-containing solution having a high concentration is preferably 100 rpm to 3,500 rpm, and more preferably 1,500 rpm to 2,700 rpm. The stirring time is preferably 1 minute to 30 minutes, and more preferably 3 minutes to 25 minutes. From the viewpoint that a high concentration of exosomes can be efficiently obtained, short-time stirring is preferable, and 1 second to 2 minutes is preferable.

It is presumed that when the bottom of the container is swirled and stirred at high speed with a vortex mixer, exosomes can be efficiently extracted from the disrupted cells, leading to the production of an exosome-containing solution having a high concentration. The temperature during the stirring treatment is not particularly limited.

The disrupted solution after the stirring treatment may be centrifuged, and the supernatant may be used in the subsequent step (filter treatment). When the disrupted solution after the stirring treatment is centrifuged, the nuclei and the like are removed from the supernatant, and clogging of the filter can be prevented, and thus the filter treatment can be efficiently performed. In the case of centrifugation, it is preferable to perform centrifugation at 100 to 1,500 × g for 3 to 10 minutes. The temperature during the centrifugal treatment is not particularly limited.

### (Step (3): step of filtering disrupted solution after stirring treatment or supernatant after centrifugation treatment)

The disrupted solution after the stirring treatment or the supernatant obtained by centrifugation is filtered through a filter. Unnecessary components can be removed by filter filtration. In addition, when a filter having an appropriate pore size is used, removal of unnecessary components and sterilizing filtration can be performed simultaneously. The material of the filter to be used for the filter treatment is not particularly limited, and cellulose acetate, which is less likely to adsorb proteins, and a filter made of metal are preferable. In particular, cellulose acetate is preferable. The filter pore size is preferably 0.1 to 0.45 µm. The filter pore size is more preferably 0.15 to 0.3 µm. When sterile filtration is also performed simultaneously, the pore size is preferably 0.2 µm. The "filter filtration" of the present invention is a physical separation method for removing unnecessary components with the force of gravity. The principle and components to be separated are different from those of centrifugal separation in which a precipitate and a supernatant are separated using centrifugal force, centrifugal acceleration, viscosity, a density difference between particles and fluid, and the like.

When the filtrate is not used immediately for therapy, it can be cryopreserved until use. Preference is given to storage at -100 to -60°C. In general, when freezing and thawing of cells are repeated, the activity of cells tends to decrease and dead cells tend to increase, but the exosome-containing solution of the present invention does not contain stem cells, and thus the quality of the exosome-containing solution does not change even when cryopreservation and thawing are repeated many times.

In the exosome-containing solution of the present invention, the solution itself can be used as a raw material without separating the exosomes, but the exosomes can also be separated and used. The method for separating exosomes from the exosome-containing solution of the present invention is not limited, and for example, the exosomes can be separated using a method such as filter filtration, gel filtration chromatography, or electrophoresis, and a combination thereof. Although the machine is expensive, exosomes may be separated from the exosome-containing solution using an ultracentrifuge.

Confirmation that the exosome obtained in the present invention is of interest can be performed by morphological observation with a transmission electron microscope, genetic analysis using PCR, immunological techniques (ELISA, FACS, or the like), Western blot, and the like.

### <Exosome-containing solution of the present invention>

The exosome-containing solution of the present invention preferably includes 10 to 10,000 pg/mL of exosomes and more preferably includes 50 to 7,500 pg/mL of exosomes when measured as the protein amount of CD63 with a CD9 × CD63 ELISA kit for quantification of human-derived exosomes manufactured by Cosmo Bio Inc. based on a cell suspension of 1 × 10⁶ cells/mL. The concentration is more preferably 75 to 5,000 pg/mL, and still more preferably 100 to 3,000 pg/mL.

According to the production method of the present invention, in addition to the exosomes secreted extracellularly, exosomes included in cells can also be efficiently extracted. Therefore, an exosome-containing solution having a high concentration can be produced. In addition, an expensive machine such as an ultracentrifuge is unnecessary and the operation is very simple, and thus the production cost can be suppressed.

In addition, the exosome-containing solution of the present invention does not include living cells, and thus the risk due to living cells such as carcinogenesis is low, there is no problem of transplant rejection, and there is no risk of causing blockage in the microvessels when the exosome-containing solution is administered as a pharmaceutical. Preparation and handling are also easy, and long-term storage by freezing is also possible. Further, the exosome-containing solution of the present invention not only contains a large amount of exosomes, but also contains a large amount of various growth factors and the like contained in cells.

### <Use of exosome-containing solution of present invention>

The exosome-containing solution obtained by the production method of the present invention can be used for various applications such as pharmaceuticals, supplements, cosmetics, and foods described later. The exosome-containing solution can be used, or the exosome can be separated from the containing solution and used. Further, it can also be used in the form of a composition by mixing an exosome-containing solution with an excipient or the like.

For example, when used as a pharmaceutical, examples thereof include a pharmaceutical containing an exosome-containing solution obtained by the production method of the present invention or separated exosomes as an active ingredient.

Exosomes are packed with intracellular components such as DNA, mRNA, miRNA, and protein, and are said to have various physiological functions. In particular, the exosome-containing solution of the present invention is effective for tissue regeneration because it is presumed that the exosome-containing solution contains not only a large amount of exosomes but also a large amount of various growth factors.

Specifically, the exosome-containing solution can be used as a therapeutic agent for, for example, central nervous system diseases such as Alzheimer's disease, Parkinson's disease, and sequelae of cerebral infarction; respiratory diseases such as interstitial lung diseases and chronic obstructive pulmonary disease; heart diseases such as myocardial infarction and heart failure; kidney disease such as nephritis (including chronic nephritis) and nephrotic syndrome; pancreatic diseases such as diabetes and pancreatitis; and inflammatory bowel diseases such as inflammatory bowel disease and Crohn's disease.

In addition, it can also be used as a therapeutic agent for a disease in which orthopedic and motor function is impaired, such as rheumatism, knee osteoarthritis, a hernia, a disease in which it is difficult to take balance of a body, and a disease in which a decrease in muscle strength is observed, such as amyotrophic lateral sclerosis.

In addition, it is presumed that the exosome-containing solution of the present invention exhibits effects such as a remarkable plumping of cumulus cells, a dominant decrease in reactive oxygen species (ROS), improvement in mitochondrial distribution and activity, a decrease in initial apoptosis in oocytes, improvement in nuclear maturation conditions and in vitro fertilization conditions, improvement in the developability of in vitro fertilized eggs, and improvement in the quality of blastocyst stage embryos. In addition, it is also said to improve the motility of sperm, and thus it can also be used for treatment and improvement of infertility due to functional deterioration of ovum and sperm, oligospermia, endometriosis, and the like, improvement of success rate of in vitro fertilization, breeding of livestock, breeding and maintenance of species (for example, maintenance of endangered species, maintenance of pet pedigree, or crossbreeding), and the like.

In addition, it is said that exosomes, due to their lipid bilayer structure, penetrate into the dermis of the skin and exert skin regeneration and anti-aging effects by proliferation and activation of fibroblasts, which are skin constituent cells, and collagen synthesis promoting effects, and thus they can also be used for anti-aging and cosmetic applications.

Alternatively, exosomes are incorporated into various tissues, and the effect of recovering tissues having deteriorated functions is also expected, and thus use as a fatigue recovery promoter is also effective. Fatigue causes reduced activity efficiency. Further, when the accumulation of fatigue becomes persistent, it can also be a factor that induces a disease. Therefore, the pharmaceutical of the present invention is also effective as a prophylactic agent that does not cause the onset of a disease.

Alternatively, the exosome-containing solution of the present invention is preferably used as a supplement for non-human mammals containing the exosome-containing solution as an active ingredient. Herein, the supplement is an enriched source of nutrients or other substances having a nutritional or physiological effect for the purpose of supplementing normal diet, and has a function useful for maintaining health and recovering from fatigue.

The pharmaceutical including the exosome-containing solution of the present invention as an active ingredient may include a non-toxic and inert pharmaceutically acceptable excipient, for example, a solid, semi-solid, or liquid diluent, a dispersant, a filler, and a carrier. Further, as long as the effect of the present invention is not impaired, a stabilizer, a preservative, a pH adjuster, a binder, a disintegrant, a surfactant, a lubricant, a flowability accelerator, a flavoring agent, a coloring agent, a perfume preservative, an antimicrobial agent, a medium, a physiological saline, an antibacterial agent, or a drug having another medicinal effect may be included as an additive.

Among these, the pharmaceutical including the exosome-containing solution of the present invention as an active ingredient preferably contains an antibacterial agent. The antibacterial agent refers to a compound and the like having an antibacterial action, and specific examples thereof include antibiotic-antimycotic solution, isopropylmethylphenol, clove oil, thymol, chlorhexidine hydrochloride, and cetylpyridinium chloride. It is preferable that these antibacterial agents are contained in an amount effective for exhibiting an antibacterial action. The pharmaceutical of the present invention includes an antibacterial agent, thereby suppressing the growth of pathogenic bacteria, and securing the safety for a certain storage period.

The dosage form of the pharmaceutical including the exosome-containing solution of the present invention as an active ingredient is not particularly limited, and may be liquid or solid, may be frozen and/or dried to be processed into a powder form, and can be variously formed into a dosage form depending on the intended use. The dosage form may be a powder, a granule, a tablet, a capsule, an injection, a drip, an inhalant, an ointment, an eye drop, a nasal drop, or a transdermal absorption agent.

The method for administering a pharmaceutical including the exosome-containing solution of the present invention as an active ingredient is not particularly limited, and may be local administration or systemic administration. Examples thereof include subcutaneous injection, intradermal injection, intramuscular injection, intranodal injection, intravenous injection, arterial injection, intraperitoneal injection, intrapleural injection, local direct injection, and direct application. In particular, in the case of intravenous injection, the agent is immediately carried to the whole body in the blood stream, resulting in a high blood concentration, and thus it is preferable to administer a pharmaceutical including the exosome-containing solution of the present invention as an active ingredient by intravenous injection when an immediate effect is required.

The dosage of the pharmaceutical including the exosome-containing solution of the present invention as an active ingredient may be any dosage as long as a therapeutic effect can be obtained, and an effective amount is administered. The level depends on the solid type, severity, age, sex, type of disease, and the like. In addition, the administration frequency is such a frequency that a therapeutic effect on a disease can be obtained when the pharmaceutical of the present invention is administered. In addition, the administration period is such a period that a therapeutic effect on a disease can be obtained when the pharmaceutical of the present invention is administered.

The administration subjects of the present invention are humans and non-human mammals for which treatment is desired or required. The non-human mammal is, for example, a monkey, a pig, a cow, a horse, a goat, a sheep, a dog, a cat, a camel, a mouse, a rat, a guinea pig, a hamster, or the like, and includes a pet animal, a livestock, and a laboratory animal.

### [Examples]

Hereinafter, the present invention will be described in detail with reference to examples, but the present invention is not limited to these examples at all.

### (1) Preparation of exosome-containing solution

### <Preparation 1 of ADSC disrupting filtrate (freeze-thawing)>

A filtrate was prepared by the method described herein using human (human A) adipose stem cells. Specifically, ADSCs were proliferated by culturing the human adipose stem cells in a MesenPRO RS Medium. The cells were subcultured for 6 passages while changing the medium every two days until 5 × 10⁶ cells were obtained. The concentration was prepared using PBS to prepare a cell suspension of 1 × 10⁶ cells/mL. This was frozen in a freezer at -20°C for 1 day and in a deep freezer at -80°C for 12 hours. Liquid nitrogen (-196°C) allows an instantaneous freeze, but herein the container including the cell suspension was immersed in liquid nitrogen for 30 minutes until the start of the measurement.

Thereafter, the frozen cell suspension was placed in a refrigerator at 4°C, and was thawed until thawing was visually confirmed. The cells were disrupted in this manner, and then subjected to a stirring treatment using a vortex mixer at a stirring time described in Table 1. The rotation speed was 2,500 rpm. Then, the liquid after the stirring treatment was filtered through a cellulose acetate membrane filter (pore size: 0.2 µm) to obtain an exosome-containing solution. Serum-free media (KBM ADSC-5, Kohjin Bio Co., Ltd.) was used as a control.

### <Quantitative analysis of exosomes by ELISA>

The amounts of exosomes in the exosome-containing solution prepared above, the culture supernatant, and the serum-free medium were measured as the amount of protein of CD63 by a CD9×CD63 ELISA kit for quantification of human-derived exosomes manufactured by Cosmo Bio Co., Ltd. The measurement method followed the protocol of the kit manufacturer. The results are shown in Table 1 and Fig. 1. Fig. 1 is a graph of the results of Table 1.

As is clear from Table 1 and Fig. 1, the exosome-containing solution obtained by stirring the freeze-thawed sample by vortex and filtering the mixture through a filter contained exosomes at a high concentration. The effect was more remarkable as the freezing temperature was lower. In particular, Example 3-3 contained as much as about 30 times as much exosomes as compared to the control (culture supernatant).

As described above, the production method of the present invention can easily produce an exosome-containing solution having a high concentration in a large amount. Use of exosomes in various applications is expected.

**[Table 1]**

| Sample name | Freezing conditions | Presence or absence and condition of vortex | Amount of exosomes included in exosome-containing solution (pg/ml) |
|---|---|---|---|
| Example 1-1 | Freezer | - | 26.8 |
| Example 1-2 | Freezer | 5 min | 70.1 |
| Example 1-3 | Freezer | 20 min | 79.1 |
| Example 2-1 | Deep freezer | - | 35.1 |
| Example 2-2 | Deep freezer | 5 min | 168.1 |
| Example 2-3 | Deep freezer | 20 min | 160.1 |
| Example 3-1 | Liquid nitrogen | | 50.2 |
| Example 3-2 | Liquid nitrogen | 5 min | 198.1 |
| Example 3-3 | Liquid nitrogen | 20 min | 237.7 |
| Culture supernatant 2 days | - | - | 8.3 |
| Media CMS | - | - | - |
| PBS(-) | - | - | - |

### <Preparation 2 of ADSC disrupting filtrate (freeze-thawing)>

Human (human B) adipose stem cells were cultured in a MesenPRO RS Medium to proliferate ADSCs. The cells were subcultured for 6 passages while changing the medium every two days until 5 × 10⁶ cells were obtained. The concentration was prepared using PBS to prepare a cell suspension of 1 × 10⁶ cells/mL. Thereafter, the same operation as in <Adjustment 1 of ADSC disrupting filtrate (freeze-thawing)> was performed while changing the operation conditions (freezing conditions, number of times of freeze-thawing, time of vortex). The operating conditions and results are shown in Table 2. When the stirring time was 1 minute or shorter, a higher concentration of exosome-containing solution was obtained. It can be said that the stirring operation for a short time is preferable because of excellent operability.

**[Table 2]**

| Sample name | Freezing conditions | Number of times of freeze-thawing (times) | Time of vortex | Amount of exosomes included in exosome-containing solution (pg/ml) |
|---|---|---|---|---|
| Example 4-1 | -20°C | 3 | 5 s | 1748.4 |
| Example 4-2 | -20°C | 3 | 10 s | 2097.9 |
| Example 4-3 | -20°C | 3 | 30 s | 2208.9 |
| Example 4-4 | -20°C | 3 | 1 min | 2105.9 |
| Example 4-5 | -20°C | 3 | 5 min | 1741.9 |
| Example 4-6 | -20°C | 3 | 10 min | 1492.4 |
| Example 5-1 | -80°C | 3 | 5 s | 2170.9 |
| Example 5-2 | -80°C | 3 | 10 s | 2178.9 |
| Example 5-3 | -80°C | 3 | 30 s | 2410.9 |
| Example 5-4 | -80°C | 3 | 1 min | 2015.4 |
| Example 5-5 | -80°C | 3 | 5 min | 1810.4 |
| Example 5-6 | -80°C | 3 | 10 min | 1315.9 |
| Example 6-1 | -196°C | 3 | 5 s | 2455.4 |
| Example 6-2 | -196°C | 3 | 10 s | 2485.9 |
| Example 6-3 | -196°C | 3 | 30 s | 2544.9 |
| Example 6-4 | -196°C | 3 | 1 min | 2320.4 |
| Example 6-5 | -196°C | 3 | 5 min | 1828.9 |
| Example 6-6 | -196°C | 3 | 10 min | 1151.9 |

### (2) Evaluation of exosome-containing solution in dog (effect on chronic nephritis)

Using canine mesenchymal stem cells (Beagle, 3 y/o male), an exosome-containing solution was prepared in the same manner as human adipose stem cells. Canine cells satisfying the following requirements were used:
- receiving combination vaccination, filaria prevention, and rabies prevention every year;
- no blood transfusion in the past;
- no systemic infectious skin disease;
- not suffering from or no suspicion of suffering from a blood-borne infection, such as babesiosis or canine brucellosis, thus far;
- no severe metabolic and endocrine diseases;
- not suffering from a genetic disease; and
- not suffering from a malignant tumor.

ADSCs of dogs satisfying the above requirements were collected, proliferated, and subcultured for 6 passages to prepare a cell suspension of 1 × 10⁶ cells/mL. Freezing was performed in a deep freezer at -80°C for 12 hours. Thereafter, the frozen cell suspension was placed in a refrigerator at 4°C, and was thawed until thawing was visually confirmed. This operation was then repeated once more. Stirring was performed at 2,500 rpm for 5 minutes with a vortex mixer, and the liquid after the stirring treatment was filtered through a cellulose acetate membrane filter (pore size: 0.2 µm) to obtain an exosome-containing solution (for dogs).

A dog suffering from chronic nephritis (Miniature Dachshund, 18 y/o female) was injected intravenously with the exosome-containing solution described above (for dogs) about every two months for a total of four times. As a result, as shown in Table 3, it was confirmed that the values of creatinine and BUN (urea nitrogen) in urine were reduced by administering the exosome-containing solution of the present invention. The concentrations of creatinine and BUN were measured using a clinical chemical analyzer for animals "FUJI DRI-CHEM NX700V".

**[Table 3]**

| Blood chemistry tests | Reference standard value | 2023/04/28 (Before administration) | 2023/12/30 (After four administrations) |
|---|---|---|---|
| BUN (urea nitrogen) | 9.2 to 29.2 mg/dL | 57.6 | 45.2 |
| Crea (creatinine) | 0.40 to 1.40 mg/dL | 2.17 | 0.60 |

### (3) Evaluation of exosome-containing solution in dog (effect on hernia)

Disc herniation is a disease in which cartilage (intervertebral disc) serving as a cushion between vertebrae in the lumbar region of the spine is degenerated, and a part of tissue protrudes, causing low back pain or causing numbness or pain in the buttocks or feet. In dogs, when disc herniation occurs, there are symptoms such as wobbling when walking, turning over of the paw, and difficulty in rising when paralysis is increased.

Herein, the exosome-containing solution (for dogs) was injected every other week for a total of four times into a vein of each of a Pomeranian (onset of lumbar disc herniation about 1 year ago) and a Miniature Dachshund (onset of disc herniation about 3 years ago).

As a result of observing the states of the dogs before and after administration of the exosome-containing solution of the present invention, it was confirmed that walking was improved, pain disappeared, and activity (energy) was improved as shown in Table 4. The evaluation shown in Table 4 is the states and activity states of the animals determined by the observer (observer and/or person who performs treatment) directly viewing the animals. A scale for measuring the quality of life of animals, called The HHHHHMM Scale, was also used for evaluation.

**[Table 4]**

| | Pomeranian (6 y/o male) | | Miniature Dachshund (12 y/o female) | |
|---|---|---|---|---|
| | Before administrat ion | After administrat ion | Before administrat ion | After administrat ion |
| Property of difficulty in walking | Difficulty in walking Jump × Difficulty in walking or decrease in waist and lateral speed Gait abnormality | Smooth walking Walking distance is extended and jump force is recovered | - | - |
| | | Walking improvement Gait improvement | | |
| Presenc e or absence of pain | With pain (Yelps when held) | Without pain (Not yelp even when held) | With pain (When held, yelps and curls back) | Without pain (Even when held, not yelp and not curl back) |
| Activity (Vitality) | Lethargic (No movement, slightly reactive to owner's movement) Loss of vitality | Shows vitality (Moving well) Improvement | - | - |
| Diarrhea symptoms | - | - | With diarrhea symptom | Without diarrhea symptom Improvement in diarrhea symptom |
| Quality of Life Scale (The HHHHHMM Scale) | HURT5 | HURT0 | HURT6 | HURT1 |

### (4) Evaluation of exosome-containing solution in dog (activity improving effect)

A Beagle dog (female 12 to 13 y/o) with the following symptoms was injected intravenously with the exosome-containing solution (for dogs) every other week for a total of four times.

As a result of observing the states of the dog before and after administration of the exosome-containing solution of the present invention, it was confirmed that walking was improved and activity (vitality) was improved as shown in Table 5. Similarly to Table 4, this is the state and activity state of the animal determined by the observer (observer and/or person who performs treatment) directly viewing the animal. This improvement is presumed to be due to the active oxygen removing action of exosomes and the action of suppressing inflammation and pain at the cellular level.

**[Table 5]**

| State before administration | State after administration |
|---|---|
| (Diagnosis result is unknown) | •Run around lightly |
| • Not interested in movement | •Quality of Life Scale (The HHHHHMM Scale) = Mobility 0 |
| • Wobble | |
| • Claudication | |
| • Plantigrade stance (wrist or heel bent at a right angle resting flat on the ground) | |
| •Quality of Life Scale (The HHHHHMM Scale) = Mobility 7 | |

### (5) Evaluation of exosome-containing solution in horse

The effect of the exosome-containing solution was also evaluated for horses. The exosome-containing solution used for the evaluation was adjusted in the same manner as the exosome-containing solution used for the test for dogs described above except that a cell suspension of 5 × 10⁶ cells/mL was used. Racehorses were intravenously injected with 1 mL of the exosome-containing solution, and the conditions of the horses one week after the administration were evaluated. The evaluation was performed by a veterinarian, and was performed according to the following criteria.

**[Table 6]**

| Evaluation criteria | Hair gloss | Tension (hardness) of muscle | Temperament |
|---|---|---|---|
| 1 | Extremely poor | Extremely poor | Extremely vicious-tempered |
| 2 | Quite poor | Quite poor | Quite vicious-tempered |
| 3 | Poor | Poor | Vicious-tempered |
| 4 | Slightly poor | Slightly poor | Slightly vicious-tempered |
| 5 | Normal | Normal | Normal |
| 6 | Slightly favorable | Slightly favorable | Slightly calm |
| 7 | Favorable | Favorable | Calm |
| 8 | Quite favorable | Quite favorable | Quite calm |
| 9 | Extremely favorable | Extremely favorable | Extremely calm |

The evaluation results are shown in Table 7. From the results, it has been also revealed that administration of the exosome-containing solution of the present invention has an effect of improving the body condition of a horse and stabilizing the mental state. Racehorses have a lot of stress due to transportation, training, and running, and the influence of stress is likely to appear on hair gloss, muscle tension, temperament, and the like, but when the exosome-containing solution of the present invention was administered, body condition and temperament were dramatically improved.

**[Table 7]**

| | Hair gloss Before administration → after administration | Tension (hardness) of muscle Before administration → after administration | Temperament Before administration → after administration |
|---|---|---|---|
| Horse 1 | 5 → 9 | 5 → 9 | 5 → 8 |
| Horse 2 | 5 → 9 | 5 → 9 | 5 → 9 |
| Horse 3 | 5 → 8 | 5 → 9 | 5 → 7 |
| Horse 4 | 5 → 9 | 5 → 9 | 5 → 9 |
| Horse 5 | 5 → 9 | 5 → 9 | 5 → 9 |
| Horse 6 | 5 → 8 | 5 → 8 | 5 → 9 |

### INDUSTRIAL APPLICABILITY

The production method of the present invention can easily produce an exosome-containing solution having a high concentration in a large amount. In addition, the pharmaceutical or supplement containing the exosome-containing solution of the present invention as an active ingredient exhibits an effect as a fatigue recovery promoter.

## Claims

1. A method for producing an exosome-containing solution derived from mesenchymal stem cells, the method comprising:
(1) a step of disrupting mesenchymal stem cells;
(2) a step of stirring the disrupted solution obtained in the step (1) using a stirrer or a vortex mixer; and
(3) a step of filtering the disrupted solution after the stirring treatment through a filter.

2. The method for producing an exosome-containing solution according to claim 1, wherein the mesenchymal stem cells are adipose tissue-derived mesenchymal stem cells.

3. The method for producing an exosome-containing solution according to claim 1 or 2, wherein the step (1) is performed by freeze-thawing or ultrasonic waves.

4. The method for producing an exosome-containing solution according to claim 1 or 2, wherein the disrupted solution is stirred at 100 to 3,500 rpm for 1 to 30 minutes using a vortex mixer in the step (2).

5. A pharmaceutical comprising, as an active ingredient, the exosome-containing solution produced by the method according to claim 1 or 2.

6. A method for promoting recovery from fatigue, wherein the pharmaceutical according to claim 5 is administered by intravenous injection.

7. A supplement for non-human mammals, comprising, as an active ingredient, the exosome-containing solution produced by the method according to claim 1 or 2.
